# EUROPEAN PATENT SPECIFICATION

(11) **EP 1 471 916 B1**
(45) Date of publication and mention of the grant of the patent: **02.07.2008**
(21) Application number: 03704474.0
(22) Date of filing: 27.01.2003
(51) Int. Cl.: A61K 31/506, A61P 19/02

(54) **TREATMENT OF RHEUMATOID ARTHRITIS USING IMATINIB**
BEHANDLUNG DER RHEUMATOIDEN ARTHRITIS MIT IMITANIB
TRAITEMENT DE LA POLYARTHRITE RHUMATOIDE AVEC IMATINIB

(30) Priority: 28.01.2002 GB 0201882
(43) Date of publication of application: 03.11.2004
(73) Proprietor: HYKS-INSTITUUTTI OY, 0029 HUS (FI)
(72) Inventor: JOENSUU, Heikki, FIN-02230 Espoo (FI)
(74) Representative: de Weerd, Petrus G.W.
(86) International application number: PCT/EP2003/000802
(87) International publication number: WO 2003/063844

(56) References cited:
- WO-A-99/03854
- WO-A-02/080925
- WO-A-02/083075
- WO-A-02/092091
- WO-A-03/002108

## Description

The invention relates to the use of 4-(4-methylpiperazin-1-ylmethyl)-N-[4-methyl-3-(4-pyridin-3-yl)pyrimidin-2-ylamino)phenyl]-benzamide (hereinafter: "COMPOUND I") in the form of its monomethanesulfonate salt for the manufacture of pharmaceutical compositions for use in the treatment of rheumatoid arthritis.

Rheumatoid Arthritis (RA) is a common chronic debilitating disease that may affect the longevity of life. The clinical course of RA is variable but it has become clear that the outcome of rheumatoid arthritis is much worse than was previously thought. The range of presentation of RA is broad but the disease onset is insidious in most cases and the first symptoms are pain, swelling and stiffness in the joints. The characteristic feature of RA is persistent inflammatory peripheral arthritis but also various extra-joint manifestations may be seen such as rheumatoid nodules, vasculitis, pulmonary fibrosis, neurological manifestations and Felty's syndrome. RA causes the loss of joint motility and can make accomplishing simple task difficult. If the persistent inflammation has led into the development of secondary amyloidosis involving kidneys, it has been estimated that the life expectancy is only about 4-5 years. RA has a substantial social effect in terms of cost, disability lost of quality of life and lost productivity. RA affects about 1% of the population in a female/male ration of 3/1. Only in the United States, about two million people suffer from RA. The disease can occur at any age but about 80% of people with RA are diagnosed between ages 35-50 and its incidence is increasing with age. The lack of test that can absolutely diagnose RA leads to a several month delay before a firm diagnosis of RA can be ascertained.

Management of RA is a major problem since there is no cure available. Therapeutic options for RA include disease modifying anti-rheumatic drugs (DMARDs). DMARDs improved inflammatory symptoms or slowed progression of joint erosions however they were often only partly effective and poorly tolerated in long-term therapy. All of the DMARDs have significant toxic side effects, which require healthcare professionals to carefully compare the risks associated with these medications versus the benefits. The methotrexate doses used in treatment of RA are small (7 to 20 mg once weekly), but they may sometimes be associated with severe adverse effects, such as hepatic fibrosis or pneumonitis. Despite 50 to 80 % of the patients undergoing the methotrexate treatment have long-term stabilization of functional status, durable remissions have never been reported. Moreover, severe RA is often treated with doses at the expense of toxicity and the response may be inadequate. Among the newly approved drugs for the treatment of RA, the so-called anti-Tumor Necrosis Factor α therapy (infliximab and etarnecept) has shown to be very effective however, approximately 30-40 % of patients do not respond even to this therapy. The cyclooxygenase (COX)-2 inhibitor (celecoxib) has a similar efficacy to classical DMARDs and a comparable safety profile but it favors cardiovascular events in patients at risk and it must be used also with caution in patients with ulcer history and in the elderly.

The utility of COMPOUND I in the context of RA alone or in combination with other agents was already mentioned in the prior art.

WO 03/002108, which was published after the filing date of the priority application of the present application, discloses the use of COMPOUND I alone or in combination with a COX-2 inhibitor, i.e. a DMARD as defined in the present application, in the treatment of RA.

WO99/03854 discloses synergistic effects of the combinations of COMPOUND I and corticosteroids. WO99/03854 teaches synergism when COMPOUND I is used in combination with anti-inflammatory drugs in the treatment of disorders arising as a result of transplantation, whereas WO99/03854 does not mention RA.

WO02/080925 discloses the concomitant use of prednisone and COMPOLTND I.

WO02/083075 discloses the use of COMPOUND I (defined as an Akt inhibitor) for the manufacture of a medicament for the treatment of RA.

WO 02/092091 discloses the combination of COMPOUND I and azathioprine, leflunomide, celecoxib and corticosteroids.

It has now surprisingly been demonstrated that RA can be successfully treated with COMPOUND I in the form of its monomethanesulfonate salt, when the monomethanesulfonate salt of COMPOUND I is administered at a daily dose corresponding to 100 to 1000 mg of COMPOUND I.

COMPOUND I is 4-(4-methylpiperazin-1-ylmethyl)-N-[4-methyl-3-(4-pyridin-3-yl)pyrimidin-2-ylamino)phenyl]-benzamide having the formula I

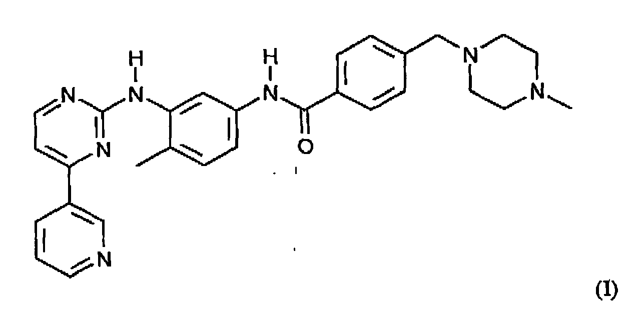

The preparation of the 4-(4-methylpiperazin-1-ylmethyl)-N-[4-methyl-3-(4-pyridin-3-yl)pyrimidin-2-ylamino)phenyl]-benzamide is described in the EP-A-0 564 409. The monomethanesulfonic acid addition salt of COMPOUND I (hereinafter "SALT I") and a preferred crystal form thereof, e.g. the beta crystal form, are described in PCT patent application WO99/03854 published on January 28, 1999.

The invention thus relates to the use of SALT I for the manufacture of pharmaceutical compositions for the treatment of rheumatoid arthritis, e.g. severe rheumatoid arthritis, DMARD-resistant rheumatoid arthritis.

In another embodiment, the invention relates to the use of the monomethanesulfonate salt, especially the beta crystal form of said salt, of COMPOUND I, for the manufacture of pharmaceutical compositions for the treatment of rheumatoid arthritis, especially severe rheumatoid arthritis.

SALT I is administered, however the dosages are expressed as the dose of COMPOUND I free base administered, e.g. for a 100 mg dose, 119.5 mg of SALT I being administered corresponding to 100 mg of COMPOUND I free base. For example, a dose of 400 mg of COMPOUND I as to be understood as 478 mg SALT I being administered corresponding to 400 mg of COMPOUND I free base.

Depending on species, age, individual condition, mode of administration, and the clinical picture in question, effective doses, for example daily doses of about 100-1000 mg, e.g. 200 to 800 mg, preferably 200-600 mg, especially 400 mg of COMPOUND I, are administered to warm-blooded animals of about 70 kg bodyweight. For adult patients with rheumatoid arthritis, a starting dose of 400 mg daily can be recommended. For patients with an inadequate response after an assessment of response to therapy with 400 mg daily, dose escalation can be safely considered and patients may be treated as long as they benefit from treatment and in the absence of limiting toxicities.

The invention relates also to a method for administering to a human subject suffering from rheumatoid arthritis, especially severe rheumatoid arthritis, COMPOUND I or a pharmaceutically acceptable salt thereof, which comprises administering a pharmaceutically effective amount of COMPOUND I or a pharmaceutically acceptable salt thereof. The invention relates especially to such method wherein a daily dose of 100 to 1000 mg, 200 to 800 mg, especially 400-600 mg, preferably 400 mg of COMPOUND I, e.g. SALT I, is administered.

The invention relates also to said method for administering to a human subject suffering from rheumatoid arthritis, especially severe rheumatoid arthritis, COMPOUND I or a pharmaceutically acceptable salt thereof, which comprises administering a pharmaceutically effective amount of COMPOUND I or a pharmaceutically acceptable salt thereof to the human subject once daily for a period exceeding 3 months.

A patient that had suffered severe RA for 30 years, her joint movements were severely limited due to the persistence of the disease. She was treated with prednisone (5 mg/day) and SALT I (orally at the dose corresponding to 600 mg then 400 mg/day of COMPOUND I free base). After several days of the treatment, she already reported improved general condition like improved joint motility and decrease of wrist swelling. After 2 and 5 months of treatment, further major improvements were noticed.

Importantly, the clinical toxicity profile of oral SALT I therapy was shown to be remarkably favorable, SALT I is relatively low toxic, and its safety profile might rival those of methotrexate or gold preparations.

### Example 1: Finding in a single patient with severe RA treated with SALT I

A patient who had concomitant rheumatoid arthritis and metastatic gastrointestinal turnor to the liver was treated with SALT I at a daily dose corresponding to 600 mg of COMPOUND I free base. The patient had suffered from RA for about 30 years, and the disease had previously been treated with multiple therapies, including gold preparations, methotrexate, NSAIDs, prednisone, and corrective surgery of the toes, feet, and wrists. The patient was diagnosed with mesangial glomerulonephritis in 1988, and she also had the diagnoses of hypertension and Crohn's disease (ileitis terminalis). Her joint movements were severely limited due to persistent RA, but she was able to walk without walking aids. SALT I was administered for nine days, discontinued for eleven days, and SALT I dosing was resumed at a daily dose corresponding to 400 mg of COMPOUND I free base on April 11. On a control visit on April 17, the patient reported improved general condition. Interestingly, she reported improved joint motility and decreased swelling particularly in the wrists, suggesting decreased activity of RA. On June 14, 2001 the patient reported further subjective improvement of her RA. For example, she was now able to comb her hair, which she was formerly unable to do due to substantially restricted shoulder movements. The upright position of her left great toe had become horizontal probably due to decrease in size of a rheumatic nodule, her wrist oedema had subsided and the wrist movements were improved. Interestingly, her occasional macroscopic hematuria had ceased suggesting improvement in RA-associated glomerulonephritis. These changes occurred despite the patient had reduced her anti-rheumatic medication. She had been taking prednisone 10 mg p.o. q.i.d. and ibuprofein 600 mg t.i.d as her anti-RA medication, but ibuprofein had been discontinued on April 4 because of the renal problems, and the patient had reduced the prednisone dose to 5 mg o.d. In September 2001, both her RA and GIST are still in remission. In January 2003, her RA is still in remission, her current medication is SALT I at a daily dose corresponding to 400 mg of COMPOUND I. All her prior RA medication have been discontinued (prednisone).

### Example 2: Clinical Trials

Three patients suffering from severe rheumatoid arthritis according to the revised American College of Rheumatology Criteria (ACR, 1987) are treated in this proof-of-concept study.

### STUDY MEDICATION

The study drug SALT I is added on the anti-rheumatic medication which the patient is taking at the onset of the study. However, those anti-rheumatic drugs which can be suspected to have drug interactions with SALT I, are discontinued before the beginning of the study. The starting dose of the drug will be 200 mg (SALT I is administered at a dose corresponding to 200 mg of COMPOUND I free base) once daily. If no severe adverse events (grade 3 or 4 according to the NCI toxicity criteria) occur the dose of SALT I will be increased to 300 mg at the beginning of the study week three and then to 400 mg once per day in the beginning of week 5, which dose is used until the end of the study unless grade 3 or 4 toxicity is encountered. If grade 3 or 4 toxicity occurs at the dose level 400 mg/day, the study medication is withheld until grade 1 or less, and then resumed at the dose of 300 mg/day, and if such toxicity occurs at the dose level 300 mg/day, SALT I is resumed at the dose of 200 mg/day. If grade 3 or 4 toxicity occurs at the dose of 200 mg/day, the patient is removed from the study.
No changes in the doses of concurrent corticosteroids, non-steroidal anti-inflammatory drugs or DMARDs is made during SALT I, medication unless the disease progresses or unless medically necessary. Intra-articular corticosteroid injections are allowed during the study if clinically indicated.

### VISIT SCHEDULE

The study is planned to last 12 weeks. During that time the patients have a screening visit (baseline) and a total of 4 follow-up visits (weeks 2, 4, 8, and 12). On each visit complete physical check up is performed (including blood pressure, pulse, body weight) and the following parameters will be assessed:
1) The number of swollen joints (in a 66 joint count)
2) The number of tender joints (in a 66 joint count)
3) VAS (visual analog pain scale)
4) The patient global assessment of disease activity
5) The doctor's global assessment of disease activity
6) HAQ (health assessment questionnaire) (weeks 0, 2, 8, 12)
7) Histological assessment of synovia from a biopsy (optional)
8) Concomitant medications
9) Adverse effects (the patients will use a diary)

*Blood tests*: At baseline (d. -7 to 0) and on each visit (visits 1 to 4) blood tests will be taken:
A. hematologogy: hemoglobin, total WBC count, a differential count including neutrophils, lymphocytes, monocytes, eosinophils and basophils
B. blood chemistry: creatinine, uric acid, albumin, total protein, total bilirubin, alkaline phophatase, AST, ALT, LDH
C. CRP, ESR and urine analysis
D. rheumatoid factor and immunoglobulins will be assessed at baseline and at 4-, 8- and 12-week visits

During the first 4 weeks of the study blood tests (A to C above) are taken weekly and during the second month of the study (weeks 4 to 8) biweekly to monitor drug safety.

*Research blood samples* are collected as at baseline, and at the 4- and 12-week visits to monitor the pro-inflammatory and anti-inflammatory cytokine levels before and during the treatment (IL-2, -6, - 10, -12, TNF-alpha, SCF).
*An X-ray of the hands* is taken before starting SALT I, (d. -7 to 0) and after 12 weeks of treatment. *Synovial fluid aspiration*. If the patient has synovitis in the knee joint which requires synovial fluid aspiration samples are be taken from the synovial fluid to determine the SCF level, mast cell tryptase level, and the activity of the synovial fluid matrix metalloproteinases.
*A synovia biopsy* may be taken at baseline within 2 weeks before starting SALT I, and repeated 2 to 3 months after enrollment (optional).

### Patients

The study is an open label, prospective study on the efficacy and safety of SALT I, in the treatment of active severe rheumatoid arthritis. Rheumatoid arthritis is required to be refractory to prior therapies including methotrexate at a dose of 10 mg or bigher per week given with a glucocorticoid, and with at least one other disease modifying anti-rheumatic drug (DMARD). Other inclusion criteria included a swollen joint count ≥ 6, and at least 2 of the following 3 findings: 1) the tender joint count ≥ 6, 2) a serum C-reactive protein (CRP) level ≥ 15 mg/l or the blood erythrocyte sedimentation rate (ESR) ≥ 28 mm/h, or 3) significant morning stiffness lasting at least for 45 minutes. The oral glucocorticoid dose is required to be 15 mg or less of prednisone or equivalent. Cytostatic drugs considered to have potential interactions with SALT I, are discontinued at least 4 weeks before the study entry. These include methotrexate and cyclophosphamide (Patient no. 3).
One male and 2 female patients had failed methotrexate with severe erosive seropositive rheumatoid arthritis resistant to DMARDs fulfilling the revised criteria of the American College of Rheumatology are enrolled (Arnett F.C. et al., 1988, Arthritis Rheum. 31:315-24). Their mean age is 56 years (range, 41 to 64). The average disease duration is 21 years (range, 19 to 23). One patient (Patient 3) is HLA-B27 positive and has a history of bilateral sacroilitis, but he has no signs of active spine disease. This patient has also secondary Sjögren's syndrome with positive speckled antinuclear antibodies.

### Administration of SALT I

SALT I is given orally as 100 mg capsules (119.5 mg of SALT I are administered and correspond to 100 mg of the free base form of COMPOUND I) with food. A dose-escalation scheme is used. The starting dose is 200 mg per day for 2 weeks given as one 100 mg capsule taken in the morning and another in the afternoon. The dose is escalated to 300 mg/day for study weeks 3 and 4, and further to 400 mg per day for the study weeks 5 to 12. The study duration is defined as 12 weeks, but the study protocol was later amended allowing those patients who benefited from SALT I, treatment to continue the treatment longer than for 12 weeks. Whenever grade 3 or 4 toxicity as defined according to the National Cancer Institute/National Institute of Health Common Toxicity Criteria (NCI/NIH-CTC) is encountered, SALT I is required to be withheld until toxicity has resolved to grade 1 or less, following which, SALT I, is restarted at a lower dose.

### Concomitant medication

No changes in the doses of concurrent corticosteroids, non-steroidal anti-inflammatory drugs or DMARDs are allowed during the study. None of the patients used corticosteroids (or equivalent) more than 10 mg/day during the study I assume you mean prednisone or equivalent. One patient (Patient 1) used oral cyclosporine 150 mg per day prior to the entry to the study, and the dose is reduced to 50 mg per day at the study entry because of the concern that SALT I might increase the blood cyclosporine concentration. However, her serum cyclosporine concentration was lower during SALT I, treatment (56 µg/l) than prior to the study (115 µg/l) when she used cyclosporine as monotherapy. Another patient (Patient 3) used hydroxychloroquine (300 mg per day) concomitantly with SALT I. Intra-articular corticostreroid injections are allowed during the study if clinically indicated. Two patients received intra-articular injections of short-acting glucocorticoids during the first 2 study months. One patient (Patient 2) had 6 joints another one (Patient 1) 2 joints injected. The injected joints were omitted from efficacy analyses.

### Outcome measures

The primary efficacy measures were: change in the number of tender and swollen joints among the 66 diarthrodial joints, patient assessed pain and disease activity as estimated using the visual analogue scales (VAS), change in the blood erythrocyte sedimentation rate (ESR) and the serum C-reactive protein (CRP) level, and change in the health assessment questionnaire (HAQ) score. The HAQ score reflects the patient functional ability (Triggiani M. et al., Int. Arch. Allergy Appl. Immunol. 1989, 88:253-5) and a high score indicates a high degree of disability. Serum CRP and blood ESR are measured prior to the study and on the study weeks 2, 4, 8, and 12. Safety assessments included examination of the vital signs every second week for the first month on study, and then monthly. A complete blood cell count is taken and renal and liver chemistry tests are performed weekly during the first 4 study weeks, and then every second week until study completion. Due to the relatively short duration of the study no serial joint radiographs are taken.

### Effect of SALT I, on outcome measures in 3 patients with severe rheumatoid arthritis.

| | Patient 1 | | Patient 2 | | Patient 3 | |
|---|---|---|---|---|---|---|
| Factor | C | w.8¹ | C | w.12 | C | w.12 |
| Swollen joint count (n) | 7 | 1 | 9 | 4 | 31 | 19 |
| Tender joint count (n) | 15 | 4 | 7 | 3 | 10 | 10 |
| Pain (VAS) (mm) | 79 | 42 | 77 | 26 | 37 | 22 |
| Patient-assessed disease activity (VAS) (mm) | 77 | 39 | 96 | 35 | 69 | 23 |
| Serum C-reactive protein (mg/l)² | 10 | 11 | 36 | 12 | 42 | 10 |
| Erythrocyte sedimentation rate (mm/h) | 38 | 23 | - | 13 | 28 | 12 |
| HAQ | 1.75 | 1.5 | 1.75 | 0.75 | 1.5 | 1.1 |
| Rheumatoid factor (IU/ml)³ | 197 | 65 | 501 | 270 | 90 | 237 |

| | | | | | | |
|---|---|---|---|---|---|---|
| ¹ SALT I treatment was interrupted on weeks 3 to 5 and discontinued on week 10; ² normal reference range < 10 mg/l; ³ normal reference range < 14 IU/ml; C: baseline; w.: week. | | | | | | |

All outcome measures improved during SALT I therapy. The swollen joint count decreased in all patients, and the tender joint count in 2 patients. The swollen and tender joint counts of Patient no. 1 increased during study weeks 3 to 5 when SALT I treatment was temporarily interrupted. The patients reported less pain and decreased disease activity on a VAS scale. Serum CRP levels decreased in 2 patients and remained unchanged in one, and the blood ESR decreased in both patients with a baseline value available. The HAQ score decreased in all patients suggesting improved functional ability. Serum rheumatoid factor levels increased initially in 2 patients (one of whom (Patient no. 3) used cyclophosphamide prior to the study), but decreased thereafter.

The clinical onset of action of SALT I is relatively fast and can be detected within 2 weeks from the start of the therapy. However, all patients reported increased joint pain during first week of treatment, which subsided below the baseline level during the second study week. Morning stiffness decreased in Patient no. 1 from 45 min to none and in Patient no. 3 from 180 min to 75 min during the study. One study subject (Patient no. 2) had a 50% clinical response to SALT I as defined by the American College of Rheumatology (ACR) criteria by the study week 12, and another patient (Patient no. 1) fulfilled the ACR 20% criteria on study week 10 when she was withdrawn from the study. Patient no. 3 failed to meet the ACR 20% criteria within the 12-week study period since his tender joint count showed no change. However, because of otherwise favorable treatment response, he preferred to continue the SALT I medication after the 12-week study period at a dose corresponding to 400 mg of COMPOUND I free base o.d., and he fulfilled the ACR 50% criteria after using SALT I, for a total of 24 weeks. The anti-rheumatic effect of SALT I, was sustained during the study period in the 2 patients who completed the 12-week study.

Rheumatoid arthritis of all 3 patients responded favorably to SALT I treatment. The swollen joint counts, serum CRP levels, and the patient-assessed disease activity and pain levels decreased, and the blood erythrocyte sedimentation rate improved. Both patients who completed the study period preferred not to discontinue SALT I, after the 12-week use of the drug. Patient no. 3 has used SALT 1 for 24 weeks, and his rheumatoid arthritis status has continued to improve with a further decrease in the number of swollen joints (from 19 at 12 weeks to 15 at 24 weeks). He also has further decrease in the duration of the morning stiffness (from 75 minutes at 12 weeks to 30 minutes at 24 weeks), decreased serum rheumatoid factor levels (237 IU/ml at 12 weeks to 139 IU/ml at 24 weeks), and bis serum CRP level and blood erythrocyte sedimentation rate have remained low (9 mg/l and 17 mm/h, respectively, at 24 weeks).

Relatively large placebo effects have commonly been observed in studies on rheumatoid arthritis, which may be explained in part by spontaneous fluctuation of the disease activity. However, the patients who participated in the present study had very active disease that was resistant to several types of antirheumatic therapies including methotrexate. The disease of patient no. 3 was also resistant to both infliximab and cyclophosphamide. In conclusion, the results of the present study suggest that SALT I could be effective in the treatment of severe rheumatoid arthritis.

### Example 3: Capsules with 4-[(4-methyl-1-piperazin-1-ylmethyl)-N-[4-methyl-3-[[4-(3-pyridinyl)-2-pyrimidinyl]amino]phenyl]benzamide monomethanesulfonate, β-crystal form

Capsules containing 119.5 mg of the compound named in the title (= SALT I) corresponding to 100 mg of COMPOUND I (free base) as active substance are prepared in the following composition:

| | | |
|---|---|---|
| Composition | SALT I | 119.5 mg |
| | Avicel | 200 mg |
| | PVPPXL | 15 mg |
| | Aerosil | 2 mg |
| | Magnesium stearate | 1.5 mg |
| | | 338.0 mg |

The capsules are prepared by mixing the components and filling the mixture into hard gelatin capsules, size 1.

## Claims

1. The use of 4-(4-methylpiperazin-1-ylmethyl)-N-[4-m3thyl-3-(4-pyridin-3-yl)pyrimidin-2-yl-amino)phenyl]-benzamide of the formula I 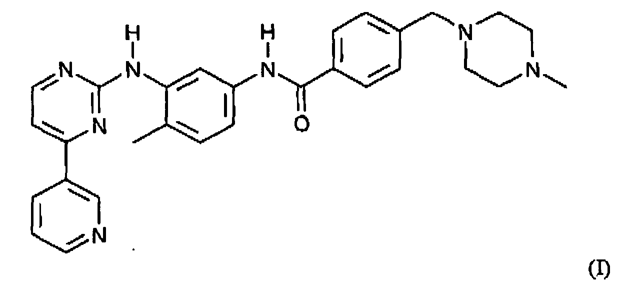 in the form of the monomethanesulfonate salt for the manufacture of a pharmaceutical composition for the treatment of rheumatoid arthritis, wherein the monomethanesulfonate salt the compound of formula I is administered at a daily dose corresponding to 100 to 1000 mg of the compound of formula I free base.

2. The use according to claim 1 **characterized in that** the rheumatoid arthritis is severe rheumatoid arthritis.

3. The use according to claim 1 **characterized in that** the rheumatoid arthritis is a disease modifying arthritis rheumatoid drug (DMARD)-resistant rheumatoid arthritis.

4. The use according to any of the preceding claims wherein the daily dose corresponds to 200 to 800 mg of the compound of formula I free base.

5. The use for according to any of the preceding claims wherein the administration is once daily for a period exceeding 3 months.

## Patentansprüche

1. Verwendung von 4-(4-Methylpiperazin-1-ylmethyl)-N-[4-methyl-3-(4-pyridin-3-yl)-pyrimidin-2-ylamino)-phenyl]-benzamid der Formel I 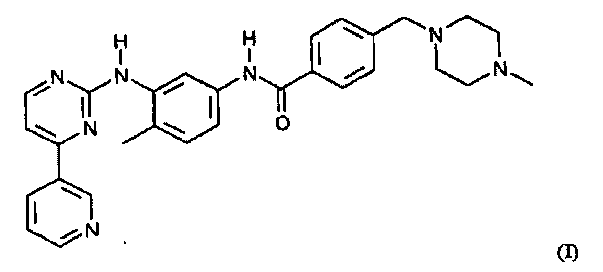 in der Form des Monomethansulfonatsalzes zur Herstellung einer pharmazeutischen Zusammensetzung für die Behandlung von rheumatoider Arthritis, wobei das Monomethansulfonatsalz der Verbindung der Formel I in einer Tagesdosis verabreicht wird, die 100 bis 1000 mg der Verbindung der Formel I als freie Base entspricht.

2. Verwendung nach Anspruch 1, **dadurch gekennzeichnet, dass** die rheumatoide Arthritis eine schwere rheumatoide Arthritis ist.

3. Verwendung nach Anspruch 1, **dadurch gekennzeichnet, dass** die rheumatoide Arthritis eine rheumatoide Arthritis mit einer Resistenz gegen ein die Krankheit modifizierendes Antirheumatikum (DMARD = Disease Modifying Anti-Rheumatic Drug) ist.

4. Verwendung nach einem der vorhergehenden Ansprüche, worin die Tagesdosis 200 bis 800 mg der Verbindung der Formel I als freie Base entspricht.

5. Verwendung nach einem der vorhergehenden Ansprüche, worin die Verabreichung einmal täglich während einer Zeitdauer entspricht, die 3 Monate übersteigt.

## Revendications

1. Utilisation de 4-(4-méthylpipérazin-1-ylméthyl)-N-[4-méthyl-3-(4-pyridin-3-yl)pyrimidin-2-yl-amino)phényl]-benzamide de formule I 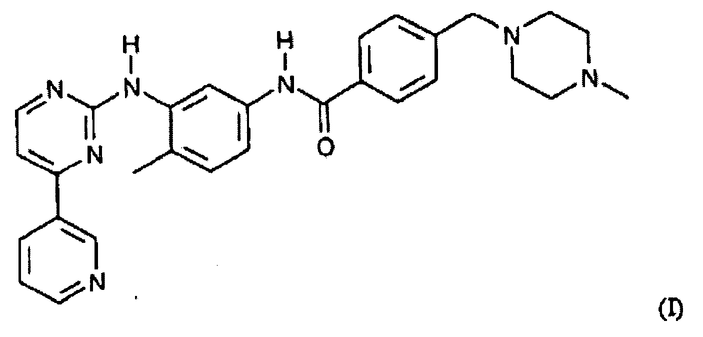 sous forme du sel monométhanesulfonate, pour fabriquer une composition pharmaceutique pour le traitement de la polyarthrite rhumatoïde, le sel monométhanesulfonate, composé de formule I, étant administré selon une dose quotidienne correspondant à 100 à 1000 mg du composé de formule I en base libre.

2. Utilisation selon la revendication 1, **caractérisée en ce que** la polyarthrite rhumatoïde est une polyarthrite rhumatoïde sévère.

3. Utilisation selon la revendication 1, **caractérisée en ce que** la polyarthrite rhumatoïde est une polyarthrite rhumatoïde qui résiste aux agents antirhumatismaux modificateurs de la maladie (DMARD).

4. Utilisation selon l'une quelconque des revendications précédentes, selon laquelle la dose quotidienne correspond à 200 à 800 mg du composé de formule I en base libre.

5. Utilisation selon l'une quelconque des revendications précédentes, selon laquelle l'administration est uniquotidienne sur une période de plus de 3 mois.
